# EUROPEAN PATENT APPLICATION

(11) **EP 1 785 091 A1**
(43) Date of publication of application: **16.05.2007**
(21) Application number: 05110713.4
(22) Date of filing: 14.11.2005
(51) Int. Cl.: A61B 6/00, G03B 42/04

(54) **Method of associating meta-data relating to a radiation image of an object with the radiation image**

(71) Applicant: Agfa HealthCare NV, 2640 Mortsel (BE)
(72) Inventor: Exelmans, Walter, 2640 Mortsel (BE)
(74) Representative: Theunis, Patrick

(57) **Abstract**

Method of associating meta-data relating to a radiation image of an object with the radiation image.

Method of associating meta-data relating to a radiation image of an object with the radiation image wherein the meta-data are broadcast and are detected and associated with an activated radiation image recording means.

## Description

### FIELD OF THE INVENTION

The present invention relates to radiography.
The invention more particularly relates to a method for associating meta data such as identification data of a patient and/or data relating to high energy radiation exposure with data representing a radiation image.

### BACKGROUND OF THE INVENTION

In addition to classical radiography systems in which a radiographic image of a patient is recorded on film, computed radiography systems and digital radiography systems are nowadays commonly used.
Computed radiography systems are for example systems that are based on storage phosphor technology.
Digital radiography systems are systems in which a radiation image is recorded on a flat panel detector such as a CMOS, a Selenium detector or the like.

In systems in which imaging is obtained by irradiation of a patient, an animal or an object with high energy radiation, it is important that the image and the meta-data giving information pertaining to the image are linked.

Meta-data are all kinds of data to be associated with the image such as demographic data (patient name, gender, date of birth etc.) and data relating to the exposure such as mAs, kV, exposure type, exposure view etc.

For example in a computed radiography system in which a radiographic image of a patient is recorded on a photostimulabale phosphor screen which is conveyed in a cassette, the meta-data are entered in a workstation or retrieved from a hospital information system or a radiology information system and transferred onto an identification means which is coupled to the cassette. Meta-data can be written into a non-volatile device for example an EEPROM device which is provided on the cassette conveying the exposed phosphor screen or the data can be transferred through radio-frequency transmission onto a radio-frequency tag provided on the cassette or on the screen.

The identified cassette conveying an exposed photo-stimulable phosphor screen is then fed into a read out apparatus (also referred to as a 'digitizer') where the meta-data are read from the identification means and where the radiographic image which is stored in the phosphor screen is read out. The radiographic image is read out by scanning the exposed photo-stimulable phosphor screen with stimulating radiation and by converting the image-wise modulated light which is emitted by the screen upon stimulation into a digital signal representation of the radiographic image.

The above-described procedure is error prone since identification and exposure are performed separate from each other so that it is possible that meta data relating to a patient and associated exposure are written into the memory device of a cassette which carries a radiation image that does not correspond with these meta data. Moreover, each separate step implies an extra time delay in the workflow.

It is also possible that the data which are written into the memory device on the cassette correspond with the intended circumstances, for example the intended or default settings of the X-ray source but which, due to various possible circumstances do not exactly represent the effectively applied exposure.

Furthermore, several prior art methods bears the risk of non-intended transfer of data to all cassettes conveying a photostimulable phosphor screen, which are present in an exposure room.

To avoid this and to make the system selective, care should be taken with the existing transfer systems that the cassette which needs to be identified is positioned within a pre-defined area. This has an limiting effect on the workflow the radiologist must follow.

It is an object of the present invention to provide a method that overcomes the above-mentioned problems associated with the prior art workflow.

### SUMMARY OF THE INVENTION

The above-mentioned aspects are realized by a method as set out in claim 1.

Specific features for preferred embodiments of the invention are set out in the dependent claims.

In the context of the present invention a "radiation image recording means" refers to a recording means capable of recording a radiation image of an object, for example a photostimulable phosphor screen,or a direct radiography image recording means whereas a "radiation detector" refers to a detector that is capable of detecting the presence of radiation.

In one embodiment the meta-data are coupled with a radiation detector by detecting and storing these data.

A radiation image recording means is said to be "activated" when it is arranged to detect and store the broadcast meta-data.
A radiation detector can be provided to detect the presence of radiation whereupon the broadcast data are detected and stored by storage means associated with the radiation image recording means with which the radiation detector is coupled. Several embodiments of applicable radiation detectors are described further on. When such a detector detects
Alternative embodiments for activating a radiation image recording means may be envisaged such as activation by pressing a button, turning a knob, turning a switch etc.

In still an alternative embodiment a request for an identifier coupled with an radiation image recording means (e.g. of a cassette conveying such a recording means) is broadcast into the radiology room. All radiation image recording means (or cassettes) present in the room will be able to receive the broadcast information.
However, only the radiation image recording means (or cassette conveying this recording means) having a radiation detector that has detected radiation, also called an activated radiation image recording means (or cassette) will be arranged to respond to the sender of the broadcast information (e.g. workstation).
Activation can be implemented on similar ways as described with regard to the first embodiment.
The radiation image recording means (or cassette) that is arranged to respond will for example respond by sending a unique cassette identification number or quadrant information (i.e. in which quadrant of the recording means recording has taken place) etc.
The unique identification number can be associated with the meta data and the image data.

Further advantages and embodiments of the present invention will become apparent from the following description.

### DETAILED DESCRIPTION OF THE INVENTION

The present invention is described below with reference to a computed radiography system in which a radiation image of a patient is recorded on a photostimulable phosphor screen which is conveyed in a cassette. On or in the cassette a radiation detector is provided.

The operator can first select the meta data (also called identification data) regarding patient and/or exposure. The operator can enter the data into a workstation or alternatively select them from a list which is for example provided by the radiography information system (RIS).

The meta-data may comprise data regarding the exposure such as kV, mAs, filtering etc., as well as data regarding exposure position such as AP, PA, LL, etc. as well as identification data regarding the patient such as name, patient identification, etc. Still other types of data are possible.

Next, the exposure of an object or a patient is started.

Once the exposure has been performed, the meta-data regarding exposure and/or patient are broadcast into the radiology room.

Different situations may be envisaged: the cassettes which are present in the room may be arranged with the ability to detect the broadcast data but only the cassette to which the data pertain will be arranged to store the data or, alternatively, only the cassette to which the data pertains will accept and store the data.
This selectivity is obtained by means of a radiation detector which generates a signal to enable acceptance and storage in storage means (EPROM, RFID tag,...) of broadcast data once it has detected radiation (high energy or secondary radiation), as will be explained further on.

Detected and stored data can be applied to a display device coupled to the cassette or the image recording means so as to display at least part of the data.

The broadcasting can start before, simultaneous with or after exposure.

As a result of the exposure procedure a radiation image of an object is recorded on the photostimulable phosphor screen.

Next, the cassette carrying the exposed photostimulable phosphor screen is fed for read out into a so-called digitizing apparatus, where the radiation image is read out. The broadcast meta data are also read out and are associated with the read out image.
The settings of the read out apparatus can be adjusted in correspondence with the read out meta data.

The exposed phosphor screen is subjected to two-dimensional scanning by means of a light beam. Upon scanning the exposed screen emits image-wise modulated light. This image-wise modulated light is collected and converted into an electric signal representation of the radiation image. The electric signal representation can then be applied to an image processing unit for further processing and/or can be applied to a hard copy recorder or to a display or archive station.

### Radiation detector

The radiation detector, in one embodiment provided in or on a cassette conveying a radiation image recording means such as a photostimulable phosphor screen, is used to activate the associated radiation image recording means, i.e. to provide the selective coupling of the broadcast meta-data to a single radiation image recording means.

The radiation detector is used to detect the presence of radiation and to trigger storage of the broadcast meta data in a storage means associated with a radiation image recording means.

It may be implemented as one of the following embodiments.
(1) In a first embodiment the secondary emission of radiation emitted by an irradiated object, is used.
   This secondary radiation emission emitted by the exposed object is guided to a transducer such as a PIN diode provided with a luminescent phosphor, detected and used to activate a receiver to receive the broadcast data.
   A PIN diode is a semiconductor that converts photons into an electric signal (X-ray photons as well as photons that are converted into visible light, e.g. by means of a phosphor).
   A color shifting fiber (scintillating fiber, a fiber provided with a phosphor layer) may be provided for collecting the primary high energy radiation and for converting it into light that can be detected by a transducer such as a PIN diode. This can be a two-step process, for example existing of a phosphor that captures X-ray photons and converts them into light within the a first (e.g. blue) wavelength range, this light of the first wavelength (blue) is then converted into light into a second (e.g. green) wavelength range which remains inside the fiber. The green light can be detected at the outer ends of the fiber by a PIN diode that converts the green light into a corresponding electric signal.
   In an alternative embodiment the secondary emission by the radiation image recording means (instead of the exposed object) e.g. in the form of light is collected and guided to a transducer such as a PIN diode.
   There are different ways to collect the light. Examples are : a fiber positioned around the radiation image recording means which captures light and guides the light towards the transducer, a plan parallel platen assembly, a reflector provided above the image detector.
(2) In an alternative embodiment the high energy radiation itself (x-rays) can be used.

The following embodiments may be envisaged:
1. a matrix of transducers (e.g. PIN diodes) positioned at the back of the radiation image recording means (side opposite to side facing the source of radiation) that detect the high energy radiation,
2. a doped fiber also positioned at the back of the detector to detect the high energy radiation, convert it into light and transport it to a transducer,
3. a transducer foil (e.g. a foil of solar cells) that detects the high energy radiation at the back of the radiation image recording means.

### Broadcast mechanism

Different energy transfer systems can be used to transfer identification meta-data. Examples are high energy radiation such as electromagnetic radiation, light waves, sound waves such as ultrasound waves etc.

### Example 1: Infrared transmission

The workstation and/or generator console sends, as soon as an X-ray irradiation has taken place, an infrared beam which is modulated by the identification data, into the exposure room.

The cassette is to be provided with an infrared transmissive window and a detector of IR rays. The detected rays are converted into electric pulses that can be interpreted as digital data.

Only the cassette that has also received an X-ray pulse will be able to accept these data.
Data will be accepted only once because e.g. storing these data will reset a flag (trigger) that has been set by the detected x-ray pulse.
In case a subsequent exposure is performed on the same cassette, the cassette can store multiple sets and sort them in time. Also the quadrant information will be added to these data.

### Example 2 : Electromagnetic waves

In this embodiment the workstation (occasionally coupled to the radiology information system) or generator console sends as soon as an X-ray irradiation has taken place, an electromagnetic wave such as a radiofrequency wave which is modulated by the identification data, into the exposure room.
Every cassette in the exposure room which is provided with an antenna will be able to detect these modulated electromagnetic waves. The detected signals can be demodulated and interpreted.
The arrangement is such that only the cassette provided with a radiation detector which has actually detected an X-ray pulse will be activated to accept the data and store the data in a storage means.
The data are only once accepted. The arrangements is such that once data are accepted, new data are ignored.

### Example 3: Ultrasound

In this case the identification data are broadcast into the exposure room by means of modulated ultrasound waves.
Every cassette in the room which is provided with a microphone will be able to detect the broadcast ultrasound wave. However, the arrangement is such that only the cassette provided with a detector that has also received an X-ray pulse will be able to accept and store the data.
The data are accepted only once. Once the data are accepted, new data are ignored.

### Triggering the exposure

A trigger is used to start the broadcast mechanism.
The following embodiments are applicable to trigger the broadcast mechanism.
1) A workstation may be coupled to the X-ray generator. As soon as the X-ray generator controls the tube to start an X-ray exposure, a signal will be send to the workstation to trigger initiation of the broadcasting of the identification data corresponding with the selected exposure.
2) In the vicinity of the x-ray tube an X-ray detector is positioned which is coupled to the workstation. Once this detector detects X-rays emitted by the tube, it will send a signal to the workstation to trigger broadcasting of the identification data corresponding with the selected exposure.

## Claims

1. Method of associating meta-data relating to a radiation image of an object with said radiation image **characterized in that** said meta-data are broadcast and are detected and associated with an activated radiation image recording means.

2. Method according to claim 1 wherein a radiation image recording means is activated when a radiation detector coupled to said radiation image recording means detects radiation whereupon storage means coupled to said radiation image recording means are triggered to store said meta-data.

3. Method according to claim 2 wherein radiation detected by said detector is high energy radiation.

4. Method according to claim 2 wherein radiation detected by said detector is secondary emission of radiation emitted by the irradiated object.

5. Method according to claim 4 wherein a scintillating fiber covered with a phosphor layer is provided for guiding said secondary emission to a transducer.

6. Method according to claim 4 wherein said secondary emission is collected and guided by means of a light guiding fiber.

7. Method according to claim 4 wherein said secondary emission is collected and guided by means of a light guiding plan parallel platen assembly.

8. Method according to claim 1 wherein high energy radiation is detected by said radiation detector.

9. Method according to claim 1 wherein said radiation image is recorded by a radiation image recording means, said radiation image recording means comprising a photostimulable phosphor screen and wherein said radiation image is read out of said photostimulable phosphor screen in a read out and digitizing apparatus.

10. Method according to claim 1 wherein said radiation image recording means is a direct radiography image recording means.

11. Method of associating meta-data relating to a radiation image of an object with said radiation image **characterized in that** a request for an identifier coupled with a radiation image recording means is broadcast, upon receipt of the broadcast request an activated radiation image recording means responds by sending said identifier, the sent identifier is associated with said meta-data.
